# EUROPEAN PATENT SPECIFICATION

(11) **EP 3 468 646 B1**
(45) Date of publication and mention of the grant of the patent: **04.03.2020**
(21) Application number: 17730928.3
(22) Date of filing: 28.04.2017
(51) Int. Cl.: A61M 5/315, A61M 5/24, A61M 5/31, A61M 5/20, A61M 5/28

(54) **TRIPLE CHAMBER WITH PREFILLED INJECTION DEVICE**
DREIFACHKAMMER MIT VORGEFÜLLTER INJEKTIONSVORRICHTUNG
CHAMBRE TRIPLE À DISPOSITIF D'INJECTION PRÉ-REMPLI

(30) Priority: 13.06.2016 IT UA20164317
(43) Date of publication of application: 17.04.2019
(73) Proprietor: Orofino Pharmaceuticals Group S.r.l., 00193 Roma (IT)
(72) Inventor: OROFINO, Ernesto, 00193 Roma (IT)
(74) Representative: Carangelo, Pierluigi
(86) International application number: PCT/IB2017/052472
(87) International publication number: WO 2017/216651

(56) References cited:
- EP-A1- 2 581 102
- EP-A2- 0 568 321
- WO-A1-97/05916
- WO-A1-2012/102216
- WO-A1-2016/012878
- JP-A- 2011 067 265

## Description

The present invention relates to a device for injecting a solution that is reconstructed immediately before being administered.

Hence the invention refers to the technical sector of injection syringes or of cartridges, in particular of the multiple chamber prefilled type.

In the pharmaceutical and medical devices sector, it is fundamental that packaging and materials contacting the active substances comply with the relative product protection and storage rules. The stability of active substances is particularly important, that is maintaining the properties of the pharmaceutical product in various temperature and relative humidity conditions, that is in different conditions of use and storage over time.

Documents EP 0568321 A2, WO 2016/012878 A1, WO 2012/102216 A1, EP 2 581 102 A1, WO 97/05916 A1 and JP 2011 067265 A disclose prior art prefilled injection devices.

Patent application number IT2014RM00408, always by this proprietor, represents an example of known syringes of the double chamber prefilled type.

Such a known syringe includes a tubular containment body (made of glass) closed at the front by a closing element (made of plastic material). As the syringe is of the prefilled type, the closing element is thus directly in contact with the active substance. Thus, in order to manufacture such a known syringe, it is first of all necessary to carry out a series of stability tests of the active substance with the material used for the closing element. Such aspect may represent a relevant disadvantage, in particular as regards the timing for placing the product into the market which is considerably slowed down due to the stability tests results.

The need perceived in the injection devices sector is to have available a device allowing to reduce the number of stability tests necessary for placing it into the market. The object of this invention is to solve the drawbacks of the prior art taking into account the needs of the sector.

Such object is obtained by an injection device according to the present invention wherein the closing element is separated and hence it is not in contact with the active substance, though the device is of the prefilled type.

The solution according to the present invention is particularly advantageous as it is necessary to carry out a limited number of stability tests of the active substance with the material used for the closing element.

The solution according to the present invention is in general suitable for injection devices, and in particular for multiple chamber injection devices of the prefilled type.

Such an object is obtained by an injection device according to claim 1. Dependent claims disclose preferred or advantageous embodiments of the device. The features and the advantages of the injection device according to the present invention are made clear from the hereinafter related exemplary and non-limiting disclosure, according to the enclosed figures, wherein:
- Figure 1 shows an axonometric view of an injection device according to the present invention;
- Figure 2 shows a cross section view of the injection device of Figure 1, in initial configuration, in one embodiment variant;
- Figure 3 shows the device of Figure 2, in pre-reconstruction configuration;
- Figure 4 shows the device of Figure 2 in final configuration;
- Figure 5 shows a cross section view of the injection device of Figure 1, in initial configuration, in one further embodiment variant;
- Figure 6 shows the device of Figure 5 in final configuration;
- Figure 7 shows a cross section view of the injection device of Figure 1, in initial configuration, in still one further embodiment variant;
- Figure 8 shows the device of Figure 7 in final configuration;
- Figure 9 shows a cross section view of the injection device of Figure 1, in initial configuration, in still one further embodiment variant;
- Figures 10 and 11 show one component of the injection device of Figure 2, and in particular the second plug provided with studs.

In the foregoing figures, equal or similar elements are indicated by the same reference numbers.

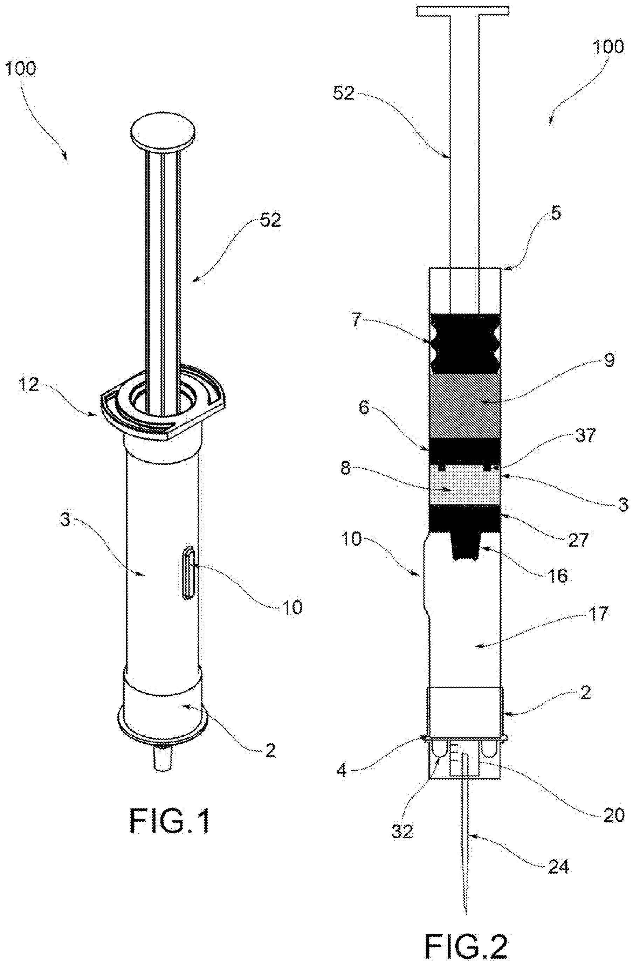

Referring to the enclosed figures, and particularly to Figure 1, an injection device is indicated by reference number 100.

The injection device 100 is prefilled and triple chamber In the embodiment represented in the foregoing Figures, the injection device is a prefilled syringe. In one alternative embodiment, the injection device 1 is a prefilled cartridge.

The prefilled injection device 100 enables to reconstruct an injectable solution immediately before its administration.

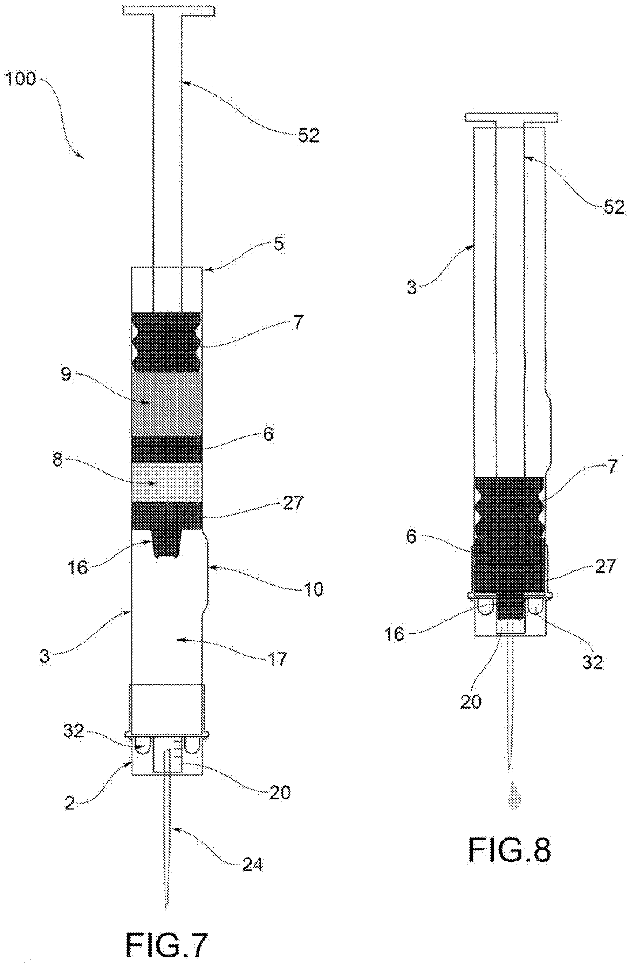

Furthermore, the embodiment shown in Figures 2,3,4,7,8,9 also allows to inject a very reduced dose of solution, for example to inject an amount of solution lower than one millilitre and preferably of, or about of 0.1 millilitre. Preferably the aforesaid dose is lower than 1/25 of the injectable solution reconstructed inside the prefilled injection device 100 and preferably equal to 1/50 of the reconstructed injectable solution.

Referring to Figure 2, the prefilled injection device 100 includes a containment tubular body 3 extending between a first opening 4, or front opening, and a second opening 5, or back opening.

The tubular body 3 is, for instance, in the form of a containment body of a syringe or of a cartridge, suitable to contain injectable substances and it is preferably made of glass or of a transparent plastic material o substantially transparent. Preferably, the tubular body 3 is made in only one piece.

The prefilled injection device 100 includes a closing element 2 fastened to the tubular body 3, e.g. fastened to the external side walls of the tubular body. The closing element 2 is fastened to an end portion of the front chamber 17.

The prefilled injection device 100 includes, at the opposite side with respect to the closing element, one ergonomic grasping portion 12, fastened to the tubular body 3 or integrally made with the tubular body 3. Preferably, the closing element 2 and/or the tubular body 3 are made of plastic material.

In the operative configuration represented in Figure 2, called "initial configuration", a first containment chamber 9, a second containment chamber 8 and a third empty chamber are defined inside the tubular body 3. In the initial configuration, the three chambers 8,9,17 are hermetically separated between them.

The first containment chamber 9 shall also be called back chamber; the second containment chamber 8 shall also be called anterior chamber; the third chamber 17 will also be called front chamber.

The prefilled injection device 100 includes, in the order (starting from the back opening 5):
- a first 7 and second 6 plug being arranged inside the tubular body 3, in order to delimitate the first containment chamber 9 therebetween in the tubular body 3, and suitable to slide inside the tubular body 3(for example, due to a pushing or traction force);
- a first liquid substance, contained in the first containment chamber 9;
- a front plug 27 being arranged inside the tubular body 3 after the second plug 6 in order to delimitate the second containment chamber 8 therewith in the tubular body 3 and suitable to slide inside the tubular body 2, (for example due to a pushing or traction force);
- a second solid or liquid substance, contained in the second containment chamber 8, intended to be mixed with the first substance inside the tubular body 3 in order to reconstruct an injectable solution;
-- an empty front chamber 17, delimited on one side by the front plug 27 and on the opposite side by the closing element 2.

Preferably, the first liquid substance is a solvent for injectable use, for example a WFI (Water for Injection) solvent or a lidocaine solution or a solution of water and benzyl alcohol or a sodium chloride physiological solution or in general any injectable substance adapted to reconstruct another solid or liquid substance. The first liquid substance may be or may contain one API (Active Pharmaceutical Ingredient).

Preferably, the second substance is a highly active substance.

The second substance is for example a powder, a substance in granules or a sterile tablet or compacted powder. The aforesaid second substance may be or may contain one API. According to one embodiment, the second substance includes two separate substances as for example two separate tablets, each containing one of said two separate substances.

If the second substance is solid, it may be a crystallized or freeze dried substance. Actually the most preferred version is the one wherein the second substance is crystallized and not freeze dried.

The aforesaid second substance is for example a highly active substance, as for example: an antibiotic, or a beta-lactam antibiotic (Cephalosporin and/or Penicillin antibiotic) or a cytotoxic anticancer agent or a hormone or a biological preparation or a biotechnological product, a monoclonal antibody, or a protein, or a vaccine, or an anaesthetic, etc. The aforesaid second substance may also be a normal active principle, that is not definable as a highly active principle.

The prefilled injection device 100 thus includes three plugs 6,7,27, in the order (starting from the back opening 5); a first plug 7 also called back plug 7, a second plug 6 also called anterior plug 6; a third plug 27 also called front plug 27.

Plugs 6,7 27 are for example made of rubber and/or plastic material and are such as to sealingly engage with the internal walls of the tubular body 3 and to slide inside the tubular body 3 under the action of an external pushing or traction force.

Preferably, the first plug 7 includes a fastening element adapted to fasten a plunger 52 to the plug so that the first plug 7 can slide inside the tubular body 3 under the pushing or traction action of the plunger 52.

The second plug 6 is spaced apart from the first plug 7 in order to delimit the first containment chamber 9. The second plug 6 is spaced apart from the front plug 27 in order to delimit the second containment chamber 8. The front plug 27 is spaced apart from the first opening 4 in order to delimit the front chamber 17.

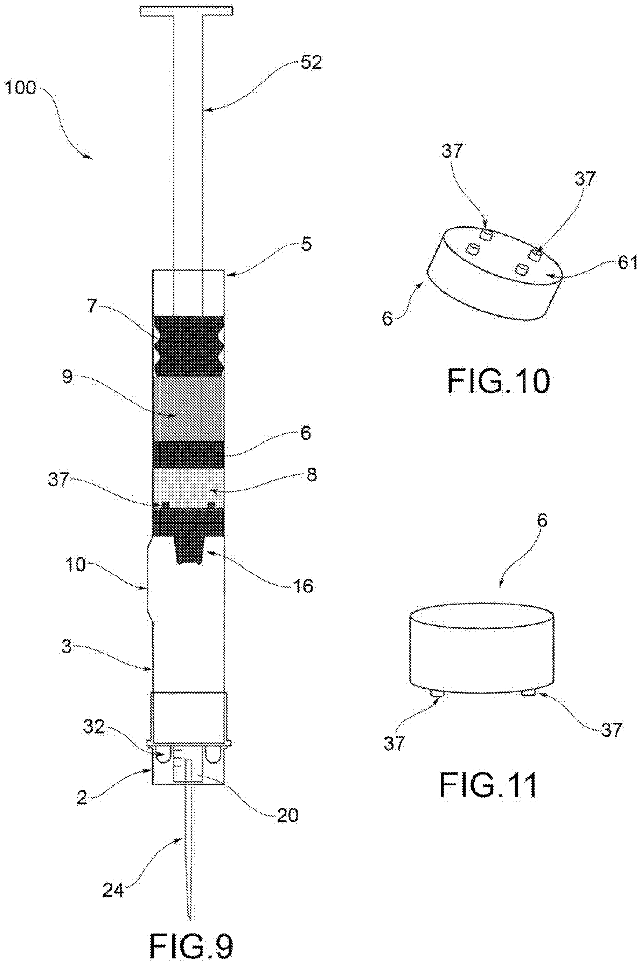

In one embodiment variant, shown in Figure 9, the front plug 27 is provided with at least one stud 37 protruding from the side facing the second chamber 8. Preferably the front plug 27 includes a plurality of studs 37, for example four studs 37.

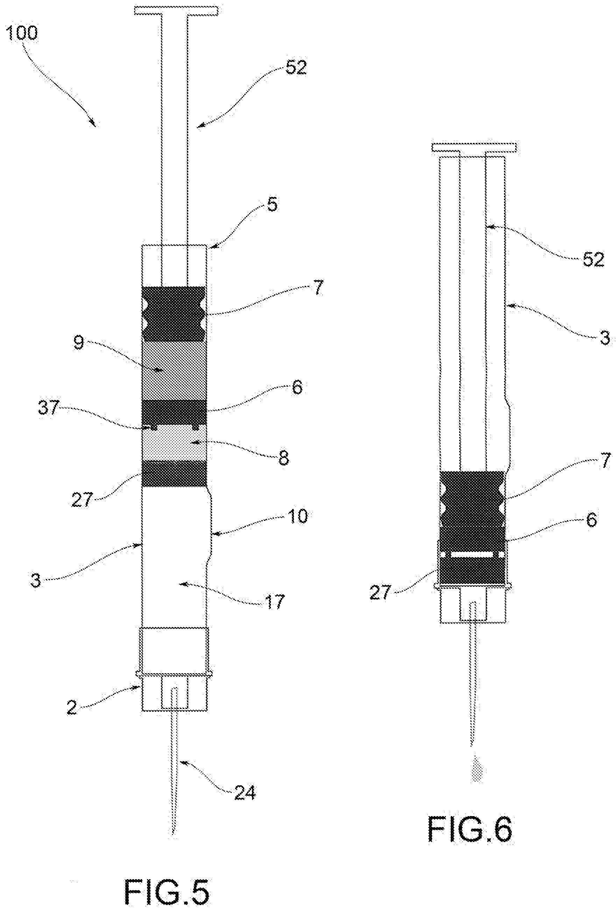

In one different embodiment variant, shown in Figures 2,5,10,11, the second plug 6 or anterior plug is provided with at least one stud 37 protruding from the side facing the second chamber 8. Preferably the second plug 6 includes a plurality of studs 37. In the example of Figure 10 the plug 6 includes four studs 37, evenly spread on the circular surface 61.

Studs 37 have for example a cylindrical, or frustoconical, or pyramidal shape.

In the embodiment shown in the Figures 2 to 9, the tubular body 3 includes an internal wall provided with a recess 10 suitable to define a bypass channel. In the initial configuration illustrated in Figure 2, such recess is positioned between the front plug 27 and the first opening 4.

The bypass channel 10 has a length LB greater than the total length LT given by summing the lengths of the front plug 27 and of the second plug 6.

In the examples of Figure 2,7,9 the length of plug 6,27 does not include the length of the protruding appendix 16.

In the examples of Figure 2,5,9 the length of plug 6,27 includes the length of studs 37.

According to one preferred embodiment, the prefilled injection device 100 includes a needle 24 whose internal channel is in communication with fluid with the opening 4. Preferably the prefilled injection device 100 includes a protective screen for the needle.

Starting from the initial configuration of Figure 2, following a pushing action by the plunger 52, for example due to a manual drive of plunger 52, plugs 6,7,27 are suitable to slide inside the tubular body 3 until it reaches a pre-reconstruction configuration wherein the second containment chamber 8 and the front chamber 17 are in communication between them thanks to bypass duct 10 and the second substance enters at least partially into the front chamber 17.

By further pushing the plunger 52 it is possible to further advance plugs 6,7 until the second plug 6 and the front plug 27 contact each other.

In the case of the embodiment variant of Figure 2,5,9 wherein the plug 6,27 includes studs 37, shaking for example manually the injection device 100 (with the closing element faced downwards) may facilitate to empty the second chamber 8 so that all the content of solution left between the plugs 6,27 is expelled.

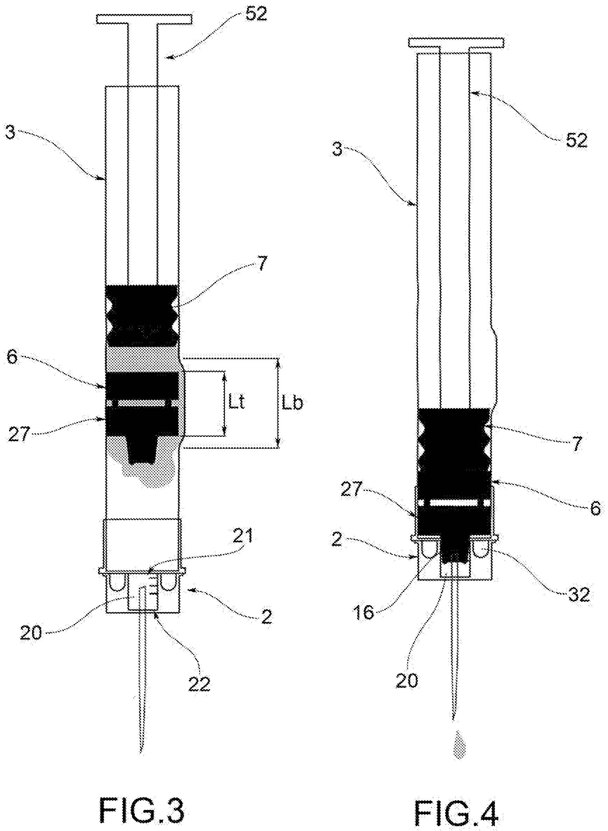

Starting from the pre-reconstruction configuration, following one further pushing action of the plunger 52, plugs 6,7,27 are suitable to further slide until they reach a so called reconstruction configuration, illustrated in Figure 3, wherein the three chambers 8, 9, 17 (or at least the first chamber 9 and the front chamber 17) are in communication with each other through the bypass duct 10 and the first substance contacts the second substance to get mixed with it. In the aforesaid reconstruction configuration, shaking for example manually the prefilled injection device 100 may facilitate the mixing of the two substances.

In the case of the embodiment variant of Figure 2,5,9 wherein the plug 6,27 includes studs 37, shaking for example manually the injection device 100 (with the closing element faced downwards) may facilitate to empty the second chamber 8 so that all the content of solution left between the plugs 6,27 is expelled.

Starting from the configuration of Fig. 3, by further pushing the plunger 52, it is possible to advance plugs 6,7,27 in order to reduce the volume of the first containment chamber 9 until when the second plug 6 and 7 contact each other, thus expelling all the content left therebeween.

By further pushing the plunger 52 it is possible to advance plugs 6,7,27 in order to reduce the volume of the front chamber 17 and expel the reconstructed solution from the tubular body 3 through the opening 4.

In the embodiment variant of Figure 2,7,9, the prefilled injection device 100 includes also a dosing reservoir 20, having an inlet opening 21 and an outlet opening 22. The inlet opening 21 is in communication with the front chamber 17 so that the front chamber 17 extends between the second plug 6 and the inlet opening 21 of the dosing reservoir 20. In the specific illustrated example, the dosing reservoir 20 is defined inside the closing element 2.

In such embodiment, under the pushing action of the plunger 52, it is possible to make the plugs 6,7,27 slide in order to reduce the volume of the front chamber 17 and expel a first fraction of the reconstructed solution from the tubular body 3, through the outlet opening 22 of the dosing reservoir 20, until a so called pre-administering configuration is reached, wherein:
- the front plug 27 obstructs the inlet opening of the dosing reservoir 20;
- one second fraction of the reconstructed solution is contained in the front chamber 17; and
- the dosing reservoir 20 is filled with a third fraction of a reconstructed solution which represents the dose of solution to administer to the patient.

Preferably, the aforesaid third fraction is lower than about 1/25 of the reconstructed injectable solution and it is preferably equal to 1/50 of the reconstructed injectable solution.

Hence, in such an example, the front plug 27 includes a protruding appendix 16 suitable to pass through the inlet opening 21 of the dosing reservoir 20 and to enter inside the latter in order to expel the third fraction of injectable solution of the dosing reservoir 20.

Preferably, the protruding appendix 16 has one cross section equal to the area of the inlet opening of the dosing reservoir 20 or slightly smaller than that in order to enter inside the dosing reservoir 20 sealingly engaging with the inner walls of the dosing reservoir 20.

In such embodiment, the prefilled injection device 100, preferably includes a venting channel provided at the closing element 2, being suitable to be passed through by a second fraction of solution in order to expel the same and to allow the protruding appendix 16 to pass through the inlet opening 21 of the dosing reservoir 20. In one alternative embodiment, a storage reservoir 32 is provided as replacement for the venting channel, suitable for passing from one closed configuration to one opened configuration following a pressure force being exerted by the frontal plug 27 on the second fraction of reconstructed solution in order to be filled with the second fraction of reconstructed solution and to enable the protruding appendix 16 to pass through the inlet opening of the dosing reservoir 20 and enter into the dosing reservoir 20.

Preferably the storage reservoir 32 is an annular reservoir.

In such embodiment variant, establishing the quantity of reconstructed solution to administer, namely the dose to be injected, occurs:
- expelling a first fraction of the reconstructed solution from the tubular body 3;
- isolating a second fraction of the reconstructed solution in the tubular body 3;
- isolating a third fraction of the reconstructed solution within the dosing reservoir 20;
wherein the second fraction is intended to be expelled through the venting channel or to be collected in the storage reservoir 32 alongside the ejection, in order to administer the third reconstructed fraction of solution from the dosing reservoir 20.

Innovatively, a prefilled injection device according to this invention needs fewer stability tests in order to be placed into the market.

Advantageously, in a prefilled injection device according to this invention the front chamber 17 is left empty and the active substance (or second substance) is bound into the middle chamber (or second chamber 8). If the contact between the active substance (or second substance) and the closing element 2 is avoided, it is not necessary to carry out any stability test of the active substance with the closing element. Thus, even though some stability tests are in any case necessary (as for example of the active substance with the material of the tubular body 3 and with the plugs material 6,7,27), the waiting time for placing the injection device on the market are significantly reduced. Furthermore, any possible change in the material used for the closing element 2 does not impact, at least not immediately, on the waiting time for placing the injection device on the market.

Advantageously, in a prefilled injection device according to this invention the presence of a plug 6,27 equipped with one or more spacer studs 37, suitable to space apart the second plug 6 from the front plug 27, enables to fully empty the second chamber 8. In fact, during operation, when the end of the second plug 6 has overcome the beginning of the bypass channel 10, the liquid substance (or first substance) enters both in the second chamber 8 and in the front chamber 17, thus contacting the active substance (or second substance) and mixing to it. By shaking the injection device 100 in this state and with the closing element 2 faced downwards, all the injectable solution is pushed into the front chamber 17.

Subject to the principle of the invention, its embodiments and its implementation details shall be widely varied with respect to what has been disclosed and illustrated for exemplary and non-limiting purposes, without detaching from the scope of protection as defined in the enclosed claims.

## Claims

1. A prefilled injection device (100), including:
- a tubular containment body (3), extending between a first opening (4) and a second opening (5), inside which a first (9) and second (8) containment chamber are defined which are hermetically divided from each other;
- a first liquid substance, being contained in the first containment chamber (9);
- a first (7) and second (6) plug being arranged inside the tubular body (3), in order to delimitate the first containment chamber (9) therebetween in the tubular body (3), and suitable to slide inside the tubular body (3);
- a second solid or liquid substance, being contained in the second containment chamber (8), intended to be mixed with the first substance inside the tubular body (3) in order to reconstruct an injectable solution;
- at least one bypass channel, being initially in a closed state and suitable to be brought in a opened state in order to reconstruct the injectable solution;
- a closing element (2) being fastened to the tubular body (3);
- a front plug (27) being arranged inside the tubular body (3) after the second plug (6) in order to delimitate the second containment chamber (8) therewith in the tubular body (3), and suitable to slide inside the tubular body (3);
- a front chamber (17), being hermetically divided from the second containment chamber (8), delimited on one side by the front plug (27) and on the opposite side by the closing element (2), said front chamber (17) being empty so that the closing element (2) is divided from the second substance;
**characterized in that**:
- the front plug (27) or the second plug (6) is provided with at least one stud (37) protruding from the side facing the second chamber (8);
- the tubular body (3) includes an internal wall being provided with a recess (10) adapted to define the bypass channel, and wherein, in an initial configuration, the bypass channel is arranged between the front plug (27) and the first opening (4).

2. The prefilled injection device (100), according to claim 1, wherein the front plug (27), or the second plug (6), is provided with a plurality of studs (37) protruding from the side facing the second chamber (8).

3. The prefilled injection device (100), according to claims 1 or 2, wherein the shape of the studs (37) is a cylindrical, frustoconical, or pyramidal one.

4. The prefilled injection device (100), according to claim 1, wherein the length (LB) of the bypass channel (10) is greater than the total length (LT) given by summing the lengths of the front plug (27) and the second plug (6), including the length of possible studs (37) and excluding the length of possible protruding appendices (16).

5. The prefilled injection device (100), according to claim 1 or 4, including a dosing reservoir (20) provided at the closing element (2), having an inlet opening (21), communicating with the front chamber (17), and an outlet opening (22), and wherein the front plug (27) includes a protruding appendix (16) adapted to pass through the inlet opening (21) of the dosing reservoir (20) to enter inside the latter in order to expel a third fraction of injectable solution from the dosing reservoir (20).

6. The prefilled injection device (100), according to claim 5, including a venting channel provided at the closing element (2), adapted to be passed through by a second fraction of solution in order to expel the same and to allow the protruding appendix (16) to pass through the inlet opening (21) of the dosing reservoir (20).

7. The prefilled injection device (100), according to claim 6, including a storage reservoir (32) provided at the closing element (2), adapted to be filled by the reconstructed second fraction of solution and to allow the protruding appendix (16) to pass through the inlet opening (21) of the dosing reservoir (20).

8. The prefilled injection device (100), according to any preceding claim, wherein the closing element (2) is made of plastic material and/or the tubular body (3) is made of glass and it is made in one piece.

9. The prefilled injection device (100), according to any preceding claim, wherein the first liquid substance is a solvent for injectable use and the second substance is an active ingredient or a highly active substance, for example an antibiotic, a beta-lactam antibiotic (Cephalosporin and/or Penicillin antibiotic), a cytotoxic anticancer agent, a hormone, a biological preparation, a biotechnological product, a monoclonal antibody, a protein, a vaccine, or an anesthetic.

## Patentansprüche

1. Eine vorgefüllte Injektionsvorrichtung (100), die Folgendes umfasst:
einen röhrenförmigen Aufnahmekörper (3), der sich zwischen einer ersten Öffnung (4) und einer zweiten Öffnung (5) erstreckt, in dem eine erste (9) und eine zweite (8) Aufnahmekammer definiert sind, die hermetisch voneinander getrennt sind;
eine erste flüssige Substanz, die in der ersten Aufnahmekammer (9) enthalten ist;
einen ersten (7) und einen zweiten (6) Stopfen, die in dem röhrenförmigen Körper (3) angeordnet sind, um die erste Aufnahmekammer (9) zwischen denselben in dem röhrenförmigen Körper (3) abzugrenzen, und die geeignet sind, in dem röhrenförmigen Körper (3) zu gleiten;
eine zweite feste oder flüssige Substanz, die in der zweiten Aufnahmekammer (8) enthalten ist, die dazu dient, mit der ersten Substanz in dem röhrenförmigen Körper (3) gemischt zu werden, um eine injizierbare Lösung zu rekonstruieren;
zumindest einen Umgehungskanal, der anfangs in einem geschlossenen Zustand ist und geeignet ist, in einen geöffneten Zustand versetzt zu werden, um die injizierbare Lösung zu rekonstruieren;
ein Schließelement (2), das an dem röhrenförmigen Körper (3) befestigt ist;
einen vorderen Stopfen (27), der in dem röhrenförmigen Körper (3) nach dem zweiten Stopfen (6) angeordnet ist, um damit die zweite Aufnahmekammer (8) in dem röhrenförmigen Körper (3) abzugrenzen, und der geeignet ist, in dem röhrenförmigen Körper (3) zu gleiten;
eine vordere Kammer (17), die von der zweiten Aufnahmekammer (8) hermetisch getrennt ist, die auf einer Seite durch den vorderen Stopfen (27) und auf der gegenüberliegenden Seite durch das Schließelement (2) abgegrenzt ist, wobei die vordere Kammer (17) leer ist, so dass das Schließelement (2) von der zweiten Substanz getrennt ist;
**dadurch gekennzeichnet, dass**:
der vordere Stopfen (27) oder der zweite Stopfen (6) mit zumindest einem Stift (37) versehen ist, der von der Seite, die der zweiten Kammer (8) zugewandt ist, vorsteht;
der röhrenförmige Körper (3) eine mit einer Ausnehmung (10) versehene Innenwand umfasst, die dahin gehend angepasst ist, den Umgehungskanal zu definieren, und wobei bei einer Anfangskonfiguration der Umgehungskanal zwischen dem vorderen Stopfen (27) und der ersten Öffnung (4) angeordnet ist.

2. Die vorgefüllte Injektionsvorrichtung (100) gemäß Anspruch 1, bei der der vordere Stopfen (27) oder der zweite Stopfen (6) mit einer Mehrzahl von Stiften (37) versehen ist, die von der Seite, die der zweiten Kammer (8) zugewandt ist, vorstehen.

3. Die vorgefüllte Injektionsvorrichtung (100) gemäß Anspruch 1 oder 2, bei der die Gestalt der Stifte (37) zylindrisch, kegelstumpfförmig oder pyramidisch ist.

4. Die vorgefüllte Injektionsvorrichtung (100) gemäß Anspruch 1, bei der die Länge (LB) des Umgehungskanals (10) größer als die Gesamtlänge (LT) ist, die sich aus der Summe der Längen des vorderen Stopfens (27) und des zweiten Stopfens (6) ergibt, einschließlich der Länge möglicher Stifte (37) und ausschließlich der Länge möglicher vorstehender Ansätze (16).

5. Die vorgefüllte Injektionsvorrichtung (100) gemäß Anspruch 1 oder 4, die einen an dem Schließelement (2) vorgesehenen Dosierbehälter (20) umfasst, der eine Einlassöffnung (21), die mit der vorderen Kammer (17) kommuniziert, und eine Auslassöffnung (22) aufweist, und wobei der vordere Stopfen (27) einen vorstehenden Ansatz (16) umfasst, der dahin gehend angepasst ist, durch die Einlassöffnung (21) des Dosierbehälters (20) hindurch zu verlaufen, um in den Letzteren einzutreten, um eine dritte Fraktion einer injizierbaren Lösung aus dem Dosierbehälter (20) auszustoßen.

6. Die vorgefüllte Injektionsvorrichtung (100) gemäß Anspruch 5, die einen an dem Schließelement (2) vorgesehenen Lüftungskanal umfasst, der dahin gehend angepasst ist, von einer zweiten Lösungsfraktion durchlaufen zu werden, um dieselbe auszustoßen und um zuzulassen, dass der vorstehende Ansatz (16) durch die Einlassöffnung (21) des Dosierbehälters (20) hindurch verläuft.

7. Die vorgefüllte Injektionsvorrichtung (100) gemäß Anspruch 6, die einen an dem Schließelement (2) vorgesehenen Speicherbehälter (32) umfasst, der dahin gehend angepasst, mit der rekonstruierten zweiten Lösungsfraktion gefüllt zu werden und zuzulassen, dass der vorstehende Ansatz (16) durch die Einlassöffnung (21) des Dosierbehälters (20) hindurch verläuft.

8. Die vorgefüllte Injektionsvorrichtung (100) gemäß einem der vorhergehenden Ansprüche, bei der das Schließelement (2) aus einem Kunststoffmaterial und/oder der röhrenförmige Körper (3) aus Glas hergestellt ist und in einem Stück hergestellt ist.

9. Die vorgefüllte Injektionsvorrichtung (100) gemäß einem der vorhergehenden Ansprüche, bei der die erste flüssige Substanz ein Lösungsmittel zur injizierbaren Verwendung und die zweite Substanz ein aktiver Inhaltsstoff oder eine hochaktive Substanz ist, beispielsweise ein Antibiotikum, ein Beta-Lactam-Antibiotikum (Cephalosporin- und/oder Penicillin-Antibiotikum), ein zytotoxischer Wirkstoff gegen Krebs, ein Hormon, eine biologische Aufbereitung, ein biotechnologisches Produkt, ein monoklonaler Antikörper, ein Protein, ein Impfstoff oder ein Anästhetikum.

## Revendications

1. Dispositif d'injection pré-rempli (100), incluant :
- un corps tubulaire (3) de confinement, s'étendant entre une première ouverture (4) et une seconde ouverture (5), à l'intérieur duquel sont définies une première (9) et une seconde chambre (8) de confinement qui sont séparées hermétiquement l'une de l'autre ;
- une première substance liquide étant confinée dans la première chambre (9) de confinement ;
- un premier (7) et un second bouchon (6) agencés à l'intérieur du corps tubulaire (3), afin de délimiter la première chambre (9) de confinement entre eux dans le corps tubulaire (3), et adaptés pour glisser à l'intérieur du corps tubulaire (3) ;
- une seconde substance solide ou liquide, étant confinée dans la seconde chambre (8) de confinement, destinée à être mélangée avec la première substance à l'intérieur du corps tubulaire (3) afin de reconstituer une solution injectable ;
- au moins un canal de dérivation étant initialement dans un état fermé et adapté pour être amené dans un état ouvert afin de reconstituer la solution injectable ;
- un élément de fermeture (2) étant fixé au corps tubulaire (3) ;
- un bouchon avant (27) étant agencé à l'intérieur du corps tubulaire (3) après le second bouchon (6) permettant de délimiter la seconde chambre (8) de confinement dans le corps tubulaire (3), et adapté pour glisser à l'intérieur du corps tubulaire (3) ;
- une chambre avant (17), étant séparée hermétiquement de la seconde chambre (8) de confinement, délimitée sur un côté par le bouchon avant (27) et sur le côté opposé par l'élément de fermeture (2), ladite chambre avant (17) étant vide de sorte que l'élément de fermeture (2) est séparé de la seconde substance ;
**caractérisé en ce que** :
- le bouchon avant (27) ou le second bouchon (6) est pourvu d'au moins un goujon (37) faisant saillie à partir du côté en regard de la seconde chambre (8) ;
- le corps tubulaire (3) inclut une paroi interne étant pourvue d'un évidement (10) adapté pour définir le canal de dérivation, et dans lequel, dans une configuration initiale, le canal de dérivation est agencé entre le bouchon avant (27) et la première ouverture (4).

2. Dispositif d'injection pré-rempli (100), selon la revendication 1, dans lequel le bouchon avant (27), ou le second bouchon (6), est pourvu d'une pluralité de goujons (37) faisant saillie à partir du côté en regard de la seconde chambre (8).

3. Dispositif d'injection pré-rempli (100), selon la revendication 1 ou 2, dans lequel la forme des goujons (37) est une forme cylindrique, tronconique ou pyramidale.

4. Dispositif d'injection pré-rempli (100), selon la revendication 1, dans lequel la longueur (LB) du canal de dérivation (10) est plus grande que la longueur totale (LT) donnée par la somme des longueurs du bouchon avant (27) et du second bouchon (6), incluant la longueur de goujons (37) éventuels et excluant la longueur d'appendices en saillie (16) éventuels.

5. Dispositif d'injection pré-rempli (100) selon la revendication 1 ou 4, incluant un réservoir de dosage (20) prévu au niveau de l'élément de fermeture (2), ayant une ouverture d'entrée (21), communiquant avec la chambre avant (17), et une ouverture de sortie (22), et dans lequel le bouchon avant (27) inclut un appendice en saillie (16) adapté pour traverser l'ouverture d'entrée (21) du réservoir de dosage (20) pour entrer à l'intérieur de ce dernier afin d'expulser une troisième fraction de solution injectable à partir du réservoir de dosage (20).

6. Dispositif d'injection pré-rempli (100) selon la revendication 5, incluant un canal de ventilation prévu au niveau de l'élément de fermeture (2), adapté pour être traversé par une deuxième fraction de solution afin d'expulser celle-ci et de permettre à l'appendice en saillie (16) de traverser l'ouverture d'entrée (21) du réservoir de dosage (20).

7. Dispositif d'injection pré-rempli (100) selon la revendication 6, incluant un réservoir de stockage (32) prévu au niveau de l'élément de fermeture (2), adapté pour être rempli par la seconde fraction reconstituée de solution et pour permettre à l'appendice en saillie (16) de traverser l'ouverture d'entrée (21) du réservoir de dosage (20).

8. Dispositif d'injection pré-rempli (100) selon une quelconque revendication précédente, dans lequel l'élément de fermeture (2) est fait en matériau plastique et/ou le corps tubulaire (3) est fait en verre et est fait d'une seule pièce.

9. Dispositif d'injection pré-rempli (100) selon une quelconque revendication précédente, dans lequel la première substance liquide est un solvant utilisé pour injection et la seconde substance est un ingrédient actif ou une substance hautement active, par exemple un antibiotique, un antibiotique bêta-lactame (antibiotique de type céphalosporine et/ou pénicilline), un agent anticancéreux cytotoxique, une hormone, une préparation biologique, un produit biotechnologique, un anticorps monoclonal, une protéine, un vaccin ou un anesthésique.
